(19) Europäisches Patentamt
European Patent Office
Office européen des brevets

(11) EP 1 431 294 A1

(12) # EUROPEAN PATENT APPLICATION
published in accordance with Art. 158(3) EPC

(43) Date of publication:
**23.06.2004 Bulletin 2004/26**

(21) Application number: **02760658.1**

(22) Date of filing: **19.08.2002**

(51) Int Cl.7: **C07D 263/58**, A61K 31/496, A61P 3/06, A61P 9/10, A61P 43/00, C07C 233/25

(86) International application number:
**PCT/JP2002/008343**

(87) International publication number:
**WO 2003/018564 (06.03.2003 Gazette 2003/10)**

(84) Designated Contracting States:
**AT BE BG CH CY CZ DE DK EE ES FI FR GB GR IE IT LI LU MC NL PT SE SK TR**
Designated Extension States:
**AL LT LV MK RO SI**

(30) Priority: **22.08.2001 JP 2001251099**

(71) Applicant: **Kowa Company Ltd.**
**Nagoya-shi Aichi-ken, 460-8625 (JP)**

(72) Inventors:
- **SHIBUYA, Kimiyuki,**
**403 Lions-Hills-Nishitokorozawa**
**Tokorozawa-shi, Saitama 359-1142 (JP)**
- **KAWAMINE, Katsumi**
**Tokorozawa-shi, Saitama 359-1145 (JP)**
- **OOGIYA, Tadaaki**
**Tokorozawa-shi, Saitama 359-1143 (JP)**
- **KITAMURA, Takahiro**
**Higashimurayama-shi, Tokyo 189-0001 (JP)**
- **MIURA, Toru**
**Higashimurayama-shi, Tokyo 189-0022 (JP)**
- **EDANO, Toshiyuki**
**Kawagoe-shi, Saitama 350-0034 (JP)**
- **YOSHINAKA, Yasunobu**
**Iruma-shi, Saitama 358-0024 (JP)**
- **YAMADA, Youichi**
**Tokorozawa-shi, Saitama 359-0038 (JP)**

(74) Representative: **Cresswell, Thomas Anthony et al**
**J.A. KEMP & CO.**
**14 South Square**
**Gray's Inn**
**London WC1R 5JJ (GB)**

(54) **VASCULAR WALL-SELECTIVE ACAT INHIBITOR**

(57) It is intended to provide a novel compound, which sustains an ACAT inhibitory activity and a macrophage-selectivity to a certain degree as a practically usable drug and can maintain an excellent oral absorption property and a favorable drug concentration in case of tested *in vivo;* a process for producing the same; medicinal compositions comprising the same and its intermediate. Namely, 2-[4-[2-(7-trifuoromethylbenzoxazol-2-ylthio)-ethyl]piperazin-1-yl]-N-(2,6-diisopropyl-4-hydroxyphenyl)-acetamide represented by the following formula: salts thereof or solvates of the same; and medicinal compositions comprising the compound represented by the above formula, salts thereof or solvates of the same together with pharmaceutically acceptable carriers.

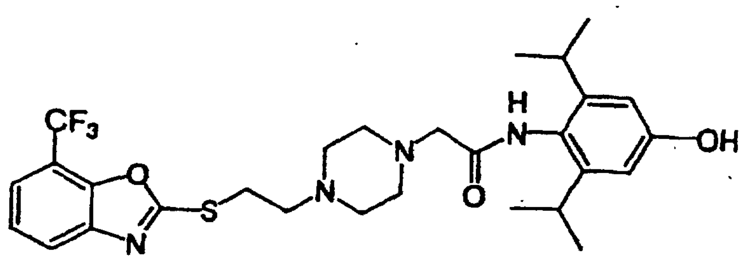



Printed by Jouve, 75001 PARIS (FR)

## Description

### Technical Field

The present invention relates to a novel compound which is useful as a drug; particularly as an acyl coenzyme A cholesterol acyltransferase (ACAT) inhibitor; more particularly as an agent for prevention, treatment and improvement of hyperlipemia and various arteriosclerotic diseases; and, still more particularly, as an agent for prevention, treatment and improvement of coronary arteriosclerotic diseases as the arteriosclerosis. It also relates to a process for producing the same, to a pharmaceutical composition using the same and to an intermediate thereof.

### Background of the Invention

In recent years, as a result of changes to dietary habit of European and American type including a high-calorie and high-cholesterol meals depending upon improvement in a level of life and also of aging of a population, there has been a rapid increase in hyperlipemia and arteriosclerotic diseases caused thereby and that is becoming one of social problems. In the drug therapy for hyperlipemia and arteriosclerosis up to now, importance has been paid mostly to lowering of lipid in blood which is a cause therefor and that is not to treat using arteriosclerotic focus per se as a target. Acyl coenzyme A: cholesterol acyltransferase (ACAT) is an enzyme which catalyzes the synthesis of cholesterol ester from cholesterol and plays an important role for metabolism and absorption in digestive organs of cholesterol. It is believed that inhibition of ACAT conducting an esterification of free cholesterol in epithelial cells of small intestine inhibits absorption of cholesterol from intestinal tract while, in liver, inhibition of production of cholesterol ester due to an ACAT inhibition suppresses secretion of very low density lipoprotein (VLDL) from liver into blood and that, as a result thereof, they bring about a lowering action for cholesterol in blood.

Many of ACAT inhibitors up to now act on ACAT of small intestine and liver as such expecting a lowering action of cholesterol in blood as a hypolipidemic agent. For example, in the specification of the U. S. Patent No. 4,716,175, 2,2-dimethyl-N-(2,4,6-trimethoxyphenyl)dodecanamide and, in the European Patent No. 372, 445, N'-(2,4-difluorophenyl)-N-[5-(4,5-diphenyl-1H-imidazol-2-ylthio)pentyl]-N-heptyl-urea, etc. are mentioned as ACAT inhibitors.

However, in many of ACAT inhibitors until now, emphasis is paid on the lowering action of cholesterol in blood as hypolipidemic agents and, during the stages of administration of large doses for expression of the action and clinical tests, side effects such as enterohemorrhage, intestinal disorder, diarrhea, hepatic disorder, etc. have frequently happened whereby clinical development has been made difficult.

Besides the above, in WO 92/09582, there is disclosed 5-[2-[2-(4-fluorophenyl)ethyl]-3-(1-methyl-1H-imidazol-2-yl)-2H-benzopyran-6-yl]oxy-2,2-dimethyl-N-(2,6-diisopropyl phenyl)pentanamide which is a compound where 2-position of imidazole is substituted and, in the European Patent No. 477,778, there is disclosed N-butyl-N'-[2-[3-(5-ethyl-4-phenylimidazol-1-yl)propoxy]-6-methylphenyl]urea which is a compound having substituents at 4 and 5-positions of imidazole. There are further disclosed the compounds such as N-(2,6-diisopropylphenyl)-2-(tetradecylthio)acetamide (refer to the specification of WO 92/09572) and N-[5-(4,5-diphenyl-1H-imidazol-2-ylthio)pentyl]-N-heptyl-2-benzoxazolamine (refer to the specification of WO 93/23392).

To begin with, arteriosclerosis is a characteristic lesion which is a thickening of inner coat of blood vessel and accumulation of lipid and, according to the recent studies, it has been believed that involution of the arteriosclerotic focus *per se* is expected by suppression of the formation of foam cells derived from macrophage which plays a central role for the formation of arteriosclerotic focus. In the atherosclerotic focus, foam cells derived from macrophage (storing cholesterol ester as lipid droplet inside the cell) are observed and it is indicated that foam cell formation of macrophage is deeply involved in the progress of the lesion. It has been also reported that ACAT activity is elevated in vascular wall of atherosclerotic lesions and that cholesterol ester is accumulated in vascular wall [Giries, P. J., et al., *Exp. Mol. Pathos*., 44, 329-339 (1986)].

Inhibition of esterification of cholesterol by an ACAT inhibitors produces free cholesterol in cells and that is eliminated by a high density lipoprotein (HDL) and carried to liver (reverse transfer by HDL) to be metabolized whereby suppression of accumulation of cholesterol ester in the lesion sites is expected [Lee, L, et al., *Biochim. Biophys. Acta*, 2001, 15, 1530 (1) : 111-22]. It is believed that, as a result, a direct anti-arteriosclerotic action is achieved. With regard toACAT, it has been reported there are two types - one existing in small intestine and another existing in vascular wall [Kinunen, P. M., et al., *Biochemistry*, 27, 7344-7350 (1988)] and many of studies for ACAT inhibitors have been carried out using an enzyme of a type which exists in small intestine and liver [Tomoda, H, et al., *J. Antibiotics*, 47, 148-153 (1994)].

The present inventors have believed that a pharmaceutical agent which selectively inhibit the ACAT which is a type of existing in vascular wall are able to be drugs for prevention, treatment and improvement of arteriosclerosis, etc. having less side effects than the inhibitors which are non-specific to organs. As a result of intensive investigations

aiming such ACAT inhibitors, the present inventors have found that a compound represented by the following formula (A) or a salt or a solvate thereof is a compound having water solubility and an excellent ACAT inhibitory action (refer to the Japanese PatentApplication No. 11/500,471 and WO 98/54153).

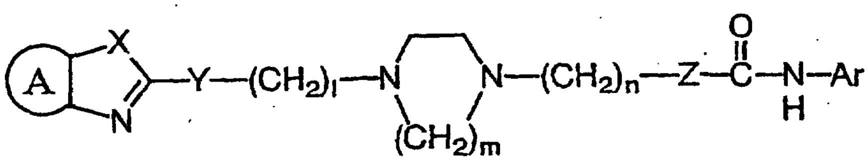 (A)

(in the formula,

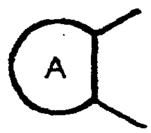

is a divalent residue of optionally substituted benzene, pyridine, cyclohexane or naphthalene or a group

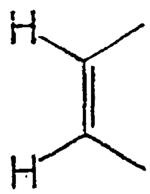

Ar is an optionally substituted aryl group,
X is -NH-, oxygen atom or sulfur atom,
Y is $-NR_1-$, oxygen atom, sulfur atom, sulfoxide or sulfone,
Z is a single bond or $-NR_2-$,
$R_1$ is hydrogen atom, a lower alkyl group, an optionally substituted aryl group or an optionally substituted silyl-lower-alkyl group,
$R_2$ is hydrogen atom, a lower alkyl group, an optionally substituted aryl group or an optionally substituted silyl-lower-alkyl group,
1 is an integer of 0 to 15,
m is an integer of 2 or 3 and
n is an integer of 0 to 3.)

With regard to the compound having an ACAT inhibitory action, not only its pharmaceutical effect but also the condition by which it is able to become a candidate for actual drug have been further investigated whereupon it is noted that the fact where only ACAT inhibitory action is excellent in an in vitro test is insufficient but it should have a good oral absorption property *per os* and, further, its excellent pharmacological effect is to be confirmed in an *in vivo* test such as animal test using appropriate arteriosclerotic models.

In view of the above, the compounds in the previous invention (Japanese Patent Application No. 11/500,471) have been further investigated and it has been found that, among the compounds specifically mentioned in the Japanese Patent Application No. 11/500,471, there is no disclosure for the compounds where, although an ACAT inhibitory activity is good, good oral absorption property is available and a sufficient drug concentration can be retained in an *in vivo* test such as an animal test using appropriate arteriosclerotic models.

As such, it is not possible from a group of the compounds specifically mentioned in the specification of the previous invention (Japanese Patent Application No. 11/500, 471) to find an excellent compound which maintains an ACAT inhibitory activity to some extent and a microphage selectivity (or a vasclular wall selectivity) and satisfies various

conditions such as oral absorption and retention of drug concentration in an *in vivo* test. With regard to the actual drugs, creation of compounds which retain an ACAT inhibitory activity and a macrophage selectivity to some extent and show an excellent oral absorption, etc. satisfies the conditions for the drugs whereby further development has been demanded.

Disclosure of the Invention

The present invention provides a novel compound which retains anACAT inhibitory activity and amacrophage selectivity to some extent as an actual drug andhas excellent oral absorption and retention of drug concentration in an in vivo test, a process for producing the same, a pharmaceutical composition using the same and an intermediate therefor.

The present invention relates to 2-[4-[2-(7-trifuoromethylbenzoxazol-2-ylthio)ethyl]piperazin-1-yl]-N-(2,6-diisopropyl-4-hydroxyphenyl)acetamide represented by the following formula, a salt thereof or a solvate thereof and to a process for producing the same.

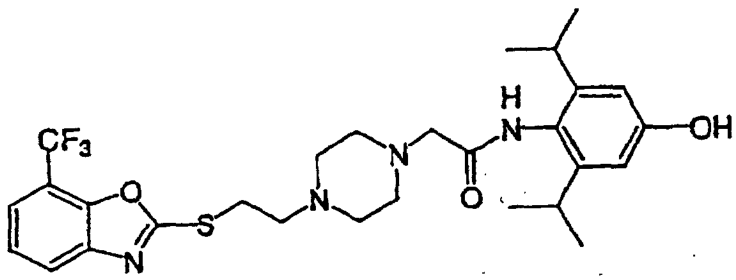

The present invention further relates to a pharmaceutical composition comprising the compound represented by the above formula, a salt thereof or a solvate thereof and a pharmaceutically acceptable carrier.

The present invention still further relates to a compound represented by the following formula, i.e. 2-halo-N-(2,6-diisopropyl-4-hydroxyphenyl)acetamide which is an intermediate for producing the compound representedby the above formula or a salt thereof.

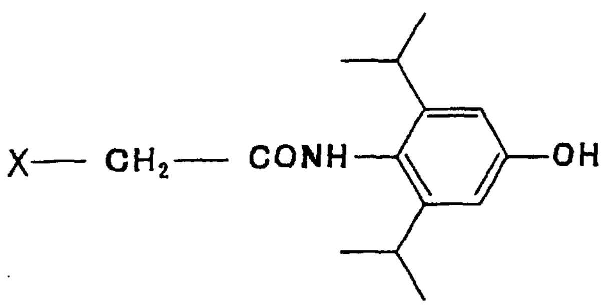

(in the formula, X is a halogen atom)

Brief Description of the Drawings

Fig. 1 is a graph which shows the result of area of lipid accumulation (white column) and total cholesterol (TC) (black column) in plasma in an animal test for the compound of the present invention using an arteriscleroticmodel. Anordinate in Fig. 1 is a relative ratio (%) to the control and an abscissa is a dose (mg/kg).

Fig. 2 is a graph which shows the result of area of lipid accumulation (white column) and total cholesterol (TC) in (black column) plasma in an animal test for the comparative compound CI-1011 using an arteriosclerotic model. An ordinate in Fig. 2 is a relative ratio (%) to the control and an abscissa is a dose (mg/kg).

Fig. 3 is a graph which shows the result of area of lipid accumulation (white column) and total cholesterol (TC)

(black column) in plasma in an animal test for the comparative compound which is a compound of Example 24 of the Japanese Patent Application No. 11/500,471 using an arteriosclerotic model. An ordinate in Fig. 3 is a relative ratio (%) to the control and an abscissa is a dose (mg/kg).

Fig. 4 is a graph which shows the relative ratio (open circle (○)) of aortic lipid accumulation to the control, relative ratio (rhombus (◊)) of total cholesterol in plasma to the control, relative ratio (black square (■)) of the ratio of cholesterol ester/free cholesterol in liver to the control, relative ratio (black triangle (▲)) of the ratio of cholesterol ester/free cholesterol in small intestine to the control and relative ratio (cross (×)) of the ratio of cholesterol ester/free cholesterol in adrenal gland to the control of the compound of the present invention in an animal test using an arteriosclerotic model. An ordinate in Fig. 4 is a relative ratio (%) to the control and an abscissa is a dose (mg/kg).

Fig. 5 is a graph which shows the relative ratio (open circle (○)) of aortic lipid accumulation to the control, relative ratio (rhombus (◊)) of total cholesterol in plasma to the control, relative ratio (black square (■)) of the ratio of cholesterol ester/free cholesterol in liver to the control, relative ratio (black triangle (▲)) of the ratio of cholesterol ester/free cholesterol in small intestine to the control and relativeratio (cross (×)) of the ratioof cholesterol ester/free cholesterol in adrenal gland to the control of the comparative compound CI-1011 in an animal test using an arteriosclerotic model. An ordinate in Fig. 5 is a relative ratio (%) to the control and an abscissa is a dose (mg/kg).

Fig. 6 is a graph which shows the relative ratio (open circle (O)) of aortic lipid accumulation to the control, relative ratio (rhombus (◊)) of total cholesterol in plasma to the control, relative ratio (black square (■)) of the ratio of cholesterol ester/free cholesterol in liver to the control, relative ratio (black triangle (▲)) of the ratio of cholesterol ester/free cholesterol in small intestine to the control and relative ratio (cross (x)) of the ratio of cholesterol ester/free cholesterol in adrenal gland to the control of the compound which is a compound of Example 24 of the Japanese Patent Application No. 11/500,471 in an animal test using an arteriosclerotic model. An ordinate in Fig. 6 is a relative ratio (%) to the control and an abscissa is a dose (mg/kg).

## Best Mode for Carrying Out the Invention

The present inventors have already provided compounds having excellent ACAT inhibitory activity and selectivity (Japanese Patent Application No. 11/500,471). Among the compound mentioned in Examples of the Japanese Patent Application No. 11/500,471, an ACAT inhibitory activity has been investigated inmore detail for the following five compounds showing an excellent ACAT inhibitory activity.

The compound of Example 24: N- [2,4-bis (methylthio) -6-methyl-3-pyridyl]-2-[4-[2-(benzoxazol-2-ylthio) ethyl]-piperazin-1-yl]acetamide

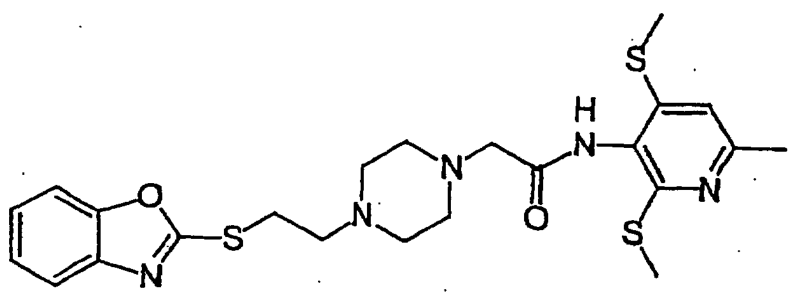

The compound of Example 25: N-[2,4-bis(methylthio)-6-methyl-3-pyridyl]-2-[4-[2-(oxazolo[4,5-b]pyridin-2-ylthio) - ethyl]-piperazin-1-yl]acetamide

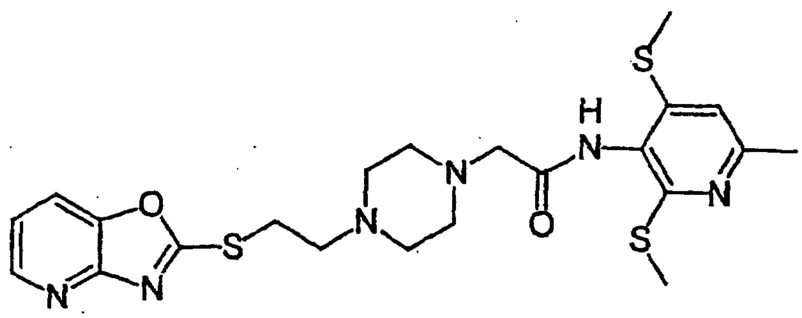

The compound of Example 75: 2-[4-[2-(benzoxazol-2-ylthio)ethyl]piperazin-1-yl]-N-(2,6-diisopropyl-4-methoxyphenyl)acetamide

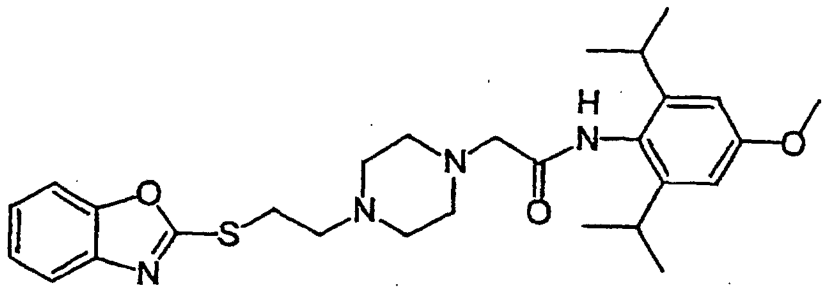

The compound of Example 78: 2-[4-[2-(benzoxazol-2-ylthio)ethyl]piperazin-1-yl]-N-[2,6-diisopropyl-4-(2-ethoxyethyl)oxyphenyl]acetamide

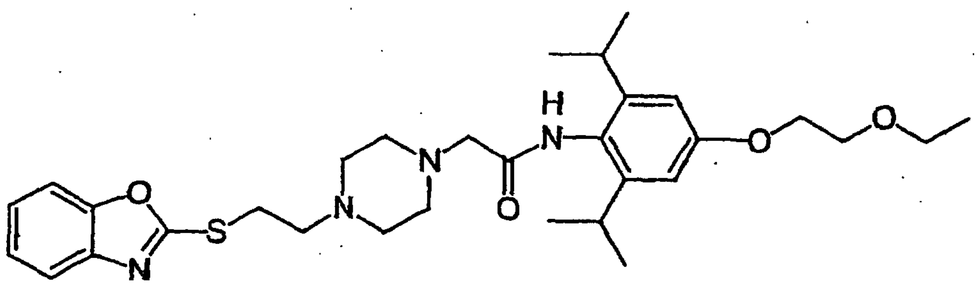

The compound of Example 85: 2-[4-[2-(7-trifluoromethylbenzoxazol-2-ylthio)ethyl]piperazin-1-yl]-N-(2,6-diisopropylphenyl)acetamide

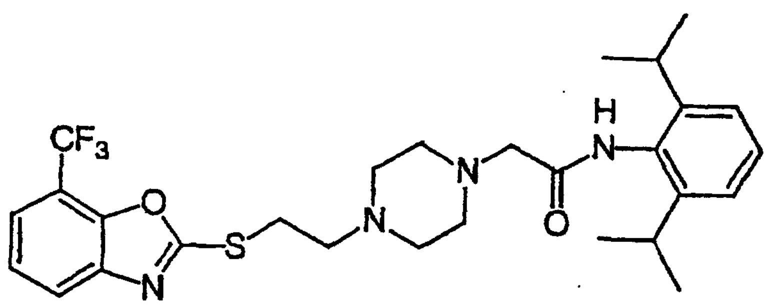

An ACAT inhibitory activity of those five compounds is mentioned in the following Table 1.

Table 1

| Tested Compounds | J774: $IC_{50}$ (nM) | HepG2: $IC_{50}$ (nM) | Ratio: HepG2/J774 |
|---|---|---|---|
| Example 24 | 9 | 60 | 7 |
| Example 25 | 100 | 9600 | 96 |
| Example 75 | 100 | 10000 | 100 |
| Example 78 | 14 | 820 | 58 |

Table 1 (continued)

| Tested Compounds | J774: $IC_{50}$ (nM) | HepG2: $IC_{50}$ (nM) | Ratio: HepG2/J774 |
|---|---|---|---|
| Example 85 | 19 | 680 | 36 |

The compound of Example 24 has the strongest ACAT inhibitory activity, particularly $IC_{50}$ in J774 cells is as high as 9 nM but its macrophage selectivity ($IC_{50}$ (HepG2)/$IC_{50}$ (J774)) is as low as about 7-fold and it is not sufficient as a candidate compound for an aimed drug.

On the other hand, the compound of Example 25 and the compound of Example 75 show such a significant action that macrophage selectivity ($IC_{50}$ (HepG2)/$IC_{50}$ (J774)) is not less than 96-fold but they are not so particularly excellent in the inhibiting intensity to J774 cells as compared with other compounds whereby they are not also sufficient as candidate compounds for aimed drugs.

Among those compounds, the compounds of Example 78 and Example 85 may be listed as the compounds in which both of' macrophage selectivity ($IC_{50}$ (HepG2) /$IC_{50}$ (J774)) and inhibitory intensity in J774 cells are satisfactory in a well balanced manner as candidate compounds for actual drugs.

When an oral absorption property was tested for those compounds of Examples 78 and 85, Cmax was 14.8 ng/mL and AUC was 29.8 ng.hr/mL in an oral absorption test by administration of 30 mg/kg for rats under a well-fed condition in the compound of Example 78 while, in the compound of Example 85, Cmax was 17.6 ng/mL and AUC was 71.8 ng.hr/mL. Those results are shown together in the following Table 2.

Table 2

| Tested Compounds | Dose | Medium for Administration | C max | AUC $_{0\text{-}inf}$ |
|---|---|---|---|---|
| Example 78 | 30 mg/kg | PEG 400:DMSO = 5:1 | 14.8 ng/mL | 29.8 ng.hr/mL |
| Example 85 | 30 mg/kg | PEG 400 | 17.6 ng/mL | 71.8 ng.hr/mL |

Although those compounds are satisfactory in terms of an ACAT inhibitory activity and a macrophage selectivity, their oral absorption and drug concentration in blood are low and are not sufficient as compounds showing a sufficient oral absorption for continuous achievement of an excellent pharmacological effect in vascular wall *in vivo*.

Therefore, in order to further investigate an effective component which shows a sufficient oral absorption by which it is able to be used as an actual drug, the present inventors have paid their attention to the compound of Example 85 which has excellent cellular ACAT inhibitory activity and macrophage selectivity. With an assumption that a compound which retains nearly the same cellular ACAT inhibitory activity in vitro and has a greatly improved oral absorption may increase the absorption rate into living body, may further enhance the bioavailability and may aim a direct arteriosclerotic therapy to the lesion site, the present inventors have investigated various derivatives using the compound as a lead compound.

As amethod for increasing the oral absorption of a compound, it is a common way of thinking to increase the water solubility of the compound and to introduce a hydrophilic group having a relatively big polarity such as hydroxyl group or carboxyl group into the lead compound for such a purpose. However, introduction of such a group having a big polarity may have a big influence on an ACAT inhibitory activity and a selectivity thereof.

Therefore, firstly, such a point has been investigated for the compounds which are specifically mentioned in the Japanese Patent Application No. 11/500,471. Thus, 2-[4-[2-(benzoxazol-2-ylthio)ethyl]piperazin-1-yl]-N-(2,6-diisopropyl-4-hydroxyphenyl) acetamide which is a compound mentioned in Example 72 of the Japanese Patent Application No. 11/500, 471 and represented by the following formula

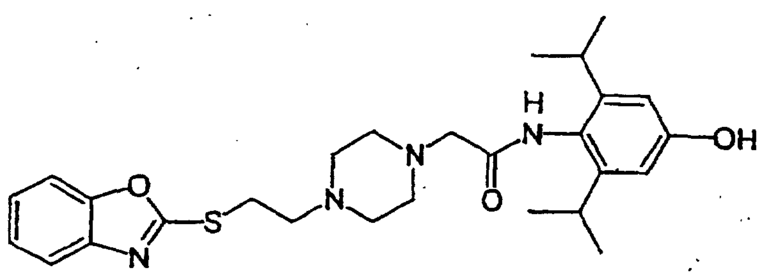

is a compound where a hydroxyl group is introduced into 4-position of a phenyl group bonding to an amide group of 2-[4-[2-(benzoxazol-2-ylthio)ethyl]piperazin-1-yl]-N-(2, 6-diisopropylphenyl) acetamide which is a compoundmentioned in Example 1 and represented by the following formula

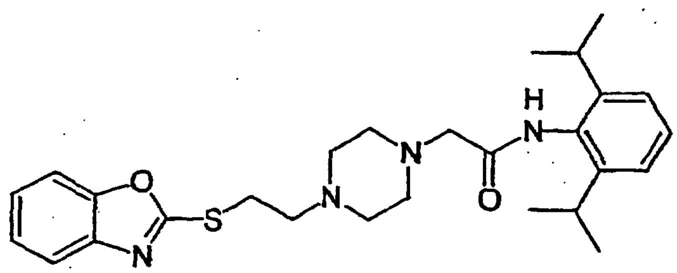

and, with regard to those compounds, their ACAT inhibitory activity and oral absorption test under a fed condition using rats (dose: 30 mg/kg) were conducted. The result is shown in the following Table 3 and Table 4.

Table 3

| Test Compounds | J774: $IC_{50}$ (nM) | HepG2: $IC_{50}$ (nM) | Ratio: HepG2/J774 |
|---|---|---|---|
| Example 1 | 260 | 680 | 3 |
| Example 72 | 130 | 780 | 7 |

Table 4

| Test Compounds | Dose | Administered Medium | $C_{max}$ | $AUC_{0\text{-}inf}$ |
|---|---|---|---|---|
| Example 1 | 30 mg/kg | 0.05N (Aq HCl) | 110 ng/mL | 295 ng.hr/mL |
| Example 72 | 30 kg/kg | 0.05N (Aq HCl) | 470 ng/mL | 1095 ng.hr/mL |

Cmax of the compound of Example 72 was 470 ng/mL and AUC was 1095 ng.hr/mL and, as compared with the original compound of Example 1, it was found that there was an improving effect in oral absorption to an extent of about 4.3-fold in terms of Cmax value (refer to Table 4). Although there was no significant change in an ACAT inhibitory activity by introduction of a hydroxyl group (refer to Table 3), any of those compounds is not able to be said an excellent ACAT inhibiting agent in view of the absolute value.

Then, in 2-[4-[2-(7-trifluoromethylbenzoxazol-2-ylthio)ethyl]piperazin-1-yl]-N-(2,6-diisopropyl-3-hydroxyphenyl)acetamide (hereinafter, referred to as a comparative compound) represented by the following formula

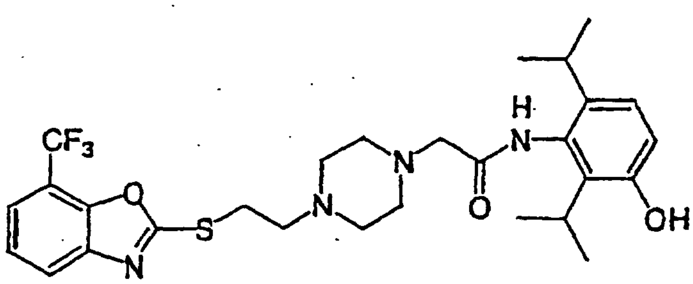

where a hydroxyl group is introduced into 3-position of a phenyl group bonding to the amide group of the compound of Example 85 in order to investigate the introduction of a hydroxyl group into the compound of Example 85 having excellent ACAT inhibitory activity and selectivity, a cellular ACAT inhibitory intensity $IC_{50}$ was 460 nM (J774) whereupon the ACAT inhibitory activity greatly lowered. The value in the compound of Example 85 which is a lead compound in the Japanese Patent Application No. 11/500,471 is 19 nM and, therefore, there was a lowering of as big as about 24-fold.

However, as a result of introduction of a hydroxyl group, improvement of water solubility was possible to some

extent and, therefore, further investigation was carried out paying our attention to the introduction of a hydroxyl group whereupon it has been unexpectedly been found that the compound of the present invention where a hydroxyl group is introduced into 4-position of the phenyl group showed excellent oral absorption and drug concentration in blood without an extreme reduction of activity.

In a hydrochloride of 2-[4-[2-(7-trifluoromethylbenzoxazol-2-ylthio)ethyl]piperazin-1-yl]-N-(2,6-diisopropyl-4-hydroxyphenyl)acetamide which is a compound of the present invention where a hydroxyl group is introduced into 4-position of a phenyl group bonding to an amide group of the compound of Example 85 which is a lead compound, an ACAT inhibitory activity test *in vitro* was.65 nM in terms of $IC_{50}$ (J774) and 2900 nM in terms of $IC_{50}$ (HepG2). Thus the selectivity ($IC_{50}$ (HepG2)/$IC_{50}$ (J774)) was about 45-fold showing more selective inhibitory activity even as compared with the lead compound without so much deterioration of inhibitory intensity in J774 cells. The result is shown in the following Table 5 together with that for the comparative compound shown already.

Table 5

| Test Compounds | J774: $IC_{50}$ (nM) | HepG2: $IC_{50}$ (nM) | Ratio: HepG2/J774 |
|---|---|---|---|
| Example 85 | 19 | 680 | 36 |
| Compound of the Invention | 65 | 2900 | 45 |
| Comparative Compound | 460 | - | - |

Further, when water solubility of the compound of the present invention was tested in the liquid I of the Japanese Pharmacopoeia, the solubility was 0.5 mg/mL as compared with that of the lead compound showing an increase of 5-fold. In an oral absorption test under a fed condition using rats (dose: 30 mg/kg), Cmax (maximum drug concentration in blood) was 141 ng/mL and AUC was 486 ng.hr/mL when the administering medium was water/PEG 400 (1: 4). The values showed about 8-fold increase in oral absorption as compared with the compound of Example 85 of the Japanese Patent Application No. 11/500,471. When the administering medium was a 0.5% methyl cellulose (MC) suspension, Cmax was 167 ng/mL and AUC was 639 ng.hr/mL and an improvement of oral absorption as high as about 9.4-fold was noted. Those results are shown in the following Table 6.

Table 6

| Test Compounds | Dose | Administering Medium | C max | $AUC_{0\text{-}inf}$ |
|---|---|---|---|---|
| Example 85 | 30 mg/kg | PEG 400 | 17.6 ng/mL | 71.8 ng.hr/mL |
| Compound of the Invention | 30 mg/kg | PEG 400: Water (4:1) | 141 ng/mL | 486 ng.hr/mL |
| Compound of the Invention | 30 mg/kg | 0.5% MC Suspension | 167 ng/mL | 639 ng.hr/mL |

On the basis of such results, the compound of the present invention was further subjected to an arteriosclerotic model animal test. The arteriosclerotic model animal test was carried out according to a method by Robert, et al. [refer to Robert, J. N., et al., *Atherosclerosis*, 137, 77-85 (1998)] in which the area where lipid was accumulated in bulbar artery in lipid-loaded model using F1B hamsters and an effect of the drug for suppression of lipid accumulation was investigated. As control drugs, the compound of Example 24 showing the strongest ACAT inhibitory activity in the Japanese Patent Application No. 11/500,471 and the compound 2,6-diisopropylphenyl N-[2-(2,4,6-triisopropylphenyl) acetyl]sulfamate which was reported already (the compound of Example 5 of the JP-T-8-510,256 (the term "JP-T" as used herein means a published Japanese translation of a PCT patent application) and WO 94/26702; hereinafter, referred to as CI-1101) were used. Result of the test is shown in the following Table 7.

Table 7

| Test Compound | Dose (mg/kg) | CE/FC | | | |
|---|---|---|---|---|---|
| | | Lipid Accumulation to Artery | Liver | Small Intestine | Adrenal Gland |
| Compound of the Invention | Control | 1.0 | 1.0 | 1.0 | 1.0 |
| | 3 | 0.69 | 1.08 | 1.16 | 1.02 |
| | 10 | 0.33 | 0.88 | 1.02 | 1.11 |
| | 30 | 0.21 | 0.60 | 0.91 | 0.89 |
| | 100 | 0.17 | 0.24 | 0.20 | 0.52 |
| Test Compound | Dose (mg/kg) | CE/FC | | | |
| | | Lipid Accumulation to Artery | Liver | Small Intestine | Adrenal Gland |
| CI-1011 | Control | 1.0 | 1.0 | 1.0 | 1.0 |
| | 0.1 | 0.73 | 1.03 | 0.92 | 0.98 |
| | 0.3 | 0.81 | 0.74 | 0.82 | 1.03 |
| | 1 | 0.35 | 0.37 | 0.60 | 0.62 |
| | 3 | 0.15 | 0.17 | 0.08 | 0.24 |
| | 10 | 0 | 0.07 | 0 | 0.28 |
| Test Compound | Dose (mg/kg) | CE/FC | | | |
| | | Lipid Accumulation to Artery | Liver | Small Intestine | Adrenal Gland |
| Compound of Example 24 | Control | 1.0 | 1.0 | 1.0 | 1.0 |
| | 1 | 0.91 | 0.89 | 0.79 | 0.6 |
| | 3 | 0.37 | 0.36 | 0.45 | 0.34 |
| | 10 | 0.32 | 0.16 | 0.21 | 0.11 |
| | 30 | 0.28 | 0.09 | 0.13 | 0.11 |

In the table, CE means cholesterol ester and FC means free cholesterol.

Those results made into graphs are shown in Fig. 1 to Fig. 6. Fig. 1 is the result of the compound of the present invention, Fig. 2 is the result of CI-1011 and Fig. 3 is the result of the compound of Example 24 of the Japanese Patent Application No. 11/500,471. Each ordinate in Fig. 1 to Fig. 3 shows a relative ratio (%) to the control while each abscissa shows dose (mg/kg) in which white column and black column show area of accumulated lipid and total cholesterol in plasma, respectively. Asterisk(s) (* or **) in Fig. 1 to Fig. 3 show(s) that a statistically significant difference is noted (*: $p < 0.05$; **: $p < 0.01$; Dunnett's test).

Fig. 4 is the result of the compound of the present invention, Fig. 5 is the result of CI-1011 and Fig. 6 is the result of the compound of Example 24 of the Japanese Patent Application No. 11/500,471. Each ordinate in Fig. 4 to Fig. 6 shows a relative ratio (%) to the control while each abscissa shows dose (mg/kg) in which each open circle (○) shows a ratio of aortic lipid accumulation, each rhombus (◊) shows a relative ratio of total cholesterol in plasma to the control, each black square (■) shows a relative ratio of the ratio of cholesterol ester/free cholesterol in liver to the control, each black triangle (▲) shows a relative ratio of the ratio of cholesterol ester/free cholesterol in small intestine to the control and each cross (x) shows a relative ratio of the ratio of cholesterol ester/free cholesterol in adrenal gland to the control.

The relative ratio is given by the following formula.

Relative Ratio (%) = (Administered (CE/FC))/(Control

(CE/FC)) × 100

From those results, the compound of the present invention was effective in suppression of lipid accumulation (white columns in Fig. 1 to Fig. 3 and white circles in Fig. 4 to Fig. 6) without a significant decrease of plasma cholesterol (black columns in Fig. 1 to Fig. 3 and rhombus in Fig. 4 to Fig. 6) when the dose was 3 mg/kg or more. On the other hand, in the case of the compound of Example 24 and the control drug (CI-1011) whereamacrophage selectivity ($IC_{50}$(HepG2)/$IC_{50}$(J774)) was low, suppression of lipid accumulation was effective as from 3 mg/kg and 1 mg/kg, respectively but all of them was accompanied by a significant decrease of plasma cholesterol whereby that was not a favorable outcome resulting in a direct action to vascular wall.

From those results, unlike' the conventional ACAT inhibitors, the compound of the present invention was found to directly and selectively inhibit the ACAT in vascular wall independently of variations in plasma lipid and to achieve suppression of accumulation of lipid on vascular wall.

The result of the *in vivo* test was also found to reflect the cell selectivity in the previous in vitro test or, in other words, a selective action of an ACAT inhibitor on the tissues of vascular wall is able to be found by investigating the lipid constitution in each tissue or the ratio of cholesterol ester (CE)/free cholesterol (FC).

There was a significant difference between the CE/FC ratio in liver, small intestine and adrenal body and the suppressing rate for accumulation of lipid to aortic arch in the doses, particularly 10 and 30 mg/kg, of the compound of the present invention and tissue selectivities of about 2.6 to 2.8-fold, 3.1 to 4.2-fold and 3.4 to 4.2-fold were noted in liver, small intestine and adrenal body, respectively. On the other hand, when the control drug (CI-1011) and the compound of Example 24 were administered, thesuppressingrateoflipidaccumulation was almost varied in accordance with the CE/FC ratio, and a significant difference of selectivities was not found in each of the tissues. Thus, unlike the conventional ACAT inhibitor which is a type acting on liver and small intestine to decrease the plasma lipid and accordingly contributing in an anti-arteriosclerotic action, the compound of the present invention is characterized as an ACAT inhibitor of a new type having a direct and selective action to vascular wall.

Thus, thepresentinventionmaterializedandaccomplished an ACAT inhibitor of a new type which is selective to vascular wall. The present invention provides a compound having an excellent ACAT inhibitory activity and the fact that a selective ACAT inhibitory action is available is particularly useful as a therapeutic agent for arteriosclerosis as a pharmaceutical composition having less side effects making the applicable range as a drug broad.

Examples of the acid addition salt of the compound of the present invention are inorganic acid salts such as hydrochloride, sulfate, nitrate and phosphate and organic acid salts such as methanesulfonate, maleate, fumarate and citrate. With regard to a solvate, there is no particular limitation so far as that a solvent used for manufacture, purification, etc. such as water or alcohol is added and that there is no bad affection to the ACAT inhibitory action, etc. A hydrate is preferred as a solvate.

The compound of the present invention maybe produced by various known processes and there is no particular limitation therefor. For example, it may be produced by a process described in the Japanese Patent Application No. 11/500,471. To be more specific, it may be produced, for example, by the following process.

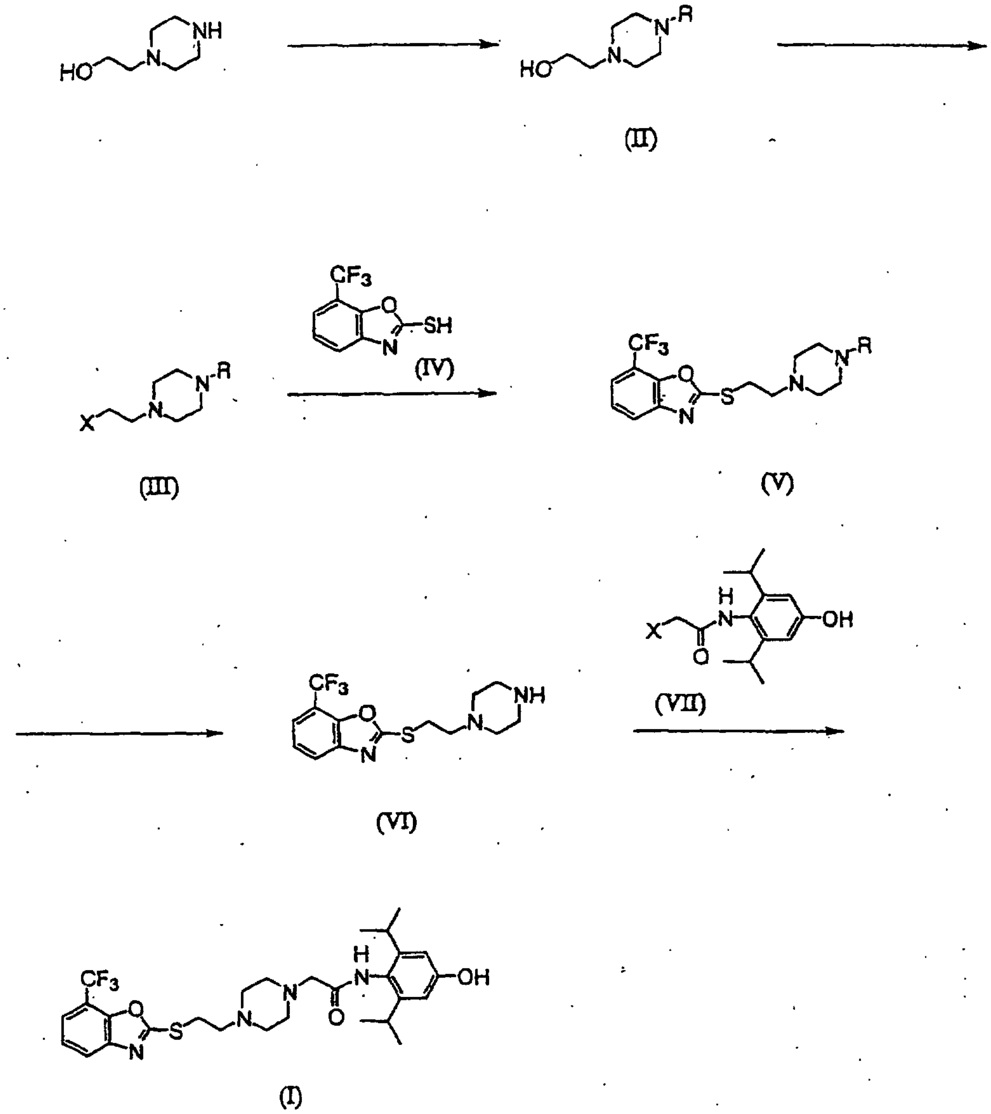

(II) (III) (IV) (V) (VI) (VII) (I)

(In the formulae, R is a protecting group and X is a leaving group.)

(1) A compound (II) where a protecting group is bonded to a nitrogen atom of 1-position of 4-(2-hydroxyethyl)-1-piperazine is converted a compound (III) where the terminal hydroxyl group is activated using methanesulfonyl chloride or the like and then that is made to react with 2-mercapto-7-trifluoromethylbenzoxazole (IV) to give the corresponding 4-[2-(7-trifluoromethylbenzoxazol-2-ylthio)ethyl]-1-piperazine where 1-position is protected (V).
(2) The protecting group of 1-position of the 1-position-protected substrate (V) prepared in the above (1) is deprotected to give 1-[2-(7-trifluoromethylbenzoxazol-2-ylthio)ethyl]piperazine or a salt thereof (VI).
(3) The compound (VI) prepared in the above (2) is made to react with 2-halo-N-(2,6-diisopropyl-4-hydroxyphenyl)-acetamide (VII) to give a desired 2-[4-[2-(7-trifluoromethylbenzoxazole-2-ylthio) ethyl] piperazin-1-yl]-N- (2, 6-diisopropyl-4-hydroxyphenyl)acetamide (I).

With regard to a protecting group for the nitrogen atom in the compound (II), various kinds of protecting groups which have been used for peptide synthesis, etc. maybe used. Examples of the preferred protecting group are benzyloxycarbonyl group, 2,2,2-trichloroethyloxycarbonyl group, 2-trimethylsilylethyloxoycarbonyl group and an alkoxycarbonyl group such as tert-butoxycarbonyl group.
When the compound (II) is subjected to a sulfonylation reaction such as mesylation or tosylation, the compound (III) is prepared. In the sulfonylation reaction, common methods may be utilized and a method using an agent for preparing sulfonate such as methanesulfonyl chloride, methanesulfonic acid anhydride, methanesulfonyl fluoride, benzenesulfonyl chloride or p-toluenesulfonyl chloride is preferred. This reaction is carried out in a solvent in the presence of a base. Examples of the solvent are tetrahydrofuran (THF), methylene chloride and chloroform. With regard to the base, an organic base such as pyridine, triethylamine, N,N-diisopropylethylamine, N-methylmorpholine and N, N-dimethylaniline may be used.

Reaction of the compound (III) with the compound (IV) is carried out in a solvent in the presence of a base.

With regard to the solvent, acetone, acetonitrile, dimethylsulfoxide, N,N-dimethylformamide, etc. are listed and, among them, N,N-dimethylformamide is preferred. With regard to the base, it is possible to use inorganic bases including alkaline metal hydroxide such as lithium hydroxide, sodium hydroxide and potassium hydroxide; alkaline metal carbonate such as sodium carbonate and potassium carbonate; metal hydrogen carbonate such as sodium hydrogen carbonate and potassium hydrogen carbonate; and also organic bases including pyridine, triethylamine and N,N-diisopropylethylamine. Potassium carbonate is particularly preferred. With regard to the reaction, it is able to be finished at 20 to 150°C for 0.1 to 20 hour(s) or, preferably, at 60 to 90°C for 1 to 5 hour(s).

Reaction for deprotection of the compound (V) may be carried out according to known methods depending upon the protecting group.

Reaction of the resulting compound (VI) with a compound (VII) may be carried out according to the above-mentioned step (1) and it is preferred that the compound (VI) is transformed an acid addition salt and then the present reaction is carried out. With regard to the acid, it is particularly preferred to use an organic acid such as acetic acid, trifluoroacetic acid or p-toluenesulfonic acid.

With regard to the halogen in the leaving group in the compound (VII) , a halogen atom such as chlorine atom or bromine atom may be listed.

The compound of the present invention has an ACAT inhibitory action and/or a intracellular cholesterol transport inhibitory action and is useful in a medical field as a therapeutic agent for hyperlipemia or a therapeutic agent for arteriosclerosis. Particularly, the compound of the present invention is expected to improve the absorption *per os* and is preferred as an effective ingredient of drugs since it shows an action of a selective inhibition of ACAT which is a type of being present in vascular wall, has less side effect as compared with non-selective ACAT inhibitors and shows a water solubility.

The pharmaceutical composition of the present invention contains the compound of the present invention, an acid addition salt thereof or a solvate thereof as an effective ingredient and comprises the effective ingredient either solely or with other pharmaceutically acceptable carriers such as excipient, binder and diluent. The pharmaceutical composition of the present invention has an pharmaceutical efficacy as an ACAT inhibitor, an intracellular cholesterol transport inhibitor, a decreasing agent for blood cholesterol or a suppressor for foaming of macrophage. Thus, the pharmaceutical composition of the present invention is useful as a pharmaceutical composition for treatment and prevention of diseases such as hyperlipemia, arteriosclerosis, neck and brain arteriosclerosis, brain vascular disorder, ischemic heart disease, coronary arteriosclerosis, nephrosclerosis, arteriosclerotic nephrosclerosis, arteriolosclerotic nephrosclerosis, malignant nephrosclerosis, ischemic intestine disease, acute mesenteric vascular obstruction, chronic intestinal tract angina, ischemic colitis, aortic aneurysm and obstructive arteriosclerosis (ASO).

The pharmaceutical composition of the present invention is able to be made into preparations such as tablets, capsules, granules, powder, injections, suppositories, etc. using various kinds of pharmaceutically acceptable carriers.

Such preparations may be manufactured by known methods. For example, in the case of a preparation for oral administration, the compound of the present invention is formulated by an appropriate combination of a diluent such as starch, mannitol and lactose; a binder such as sodium carboxymethyl cellulose and hydroxypropyl cellulose; a disintegrating agent such as crystalline cellulose and calcium carboxymethyl cellulose; a lubricant such as talc and magnesium stearate; a fluidity improving agent such as light anhydrous silicic acid; etc.

The pharmaceutical composition of the present invention is administered either orally or parenterally.

Although the dose of the pharmaceutical composition of the present invention varies depending upon body weight, age, sex, symptom, etc. of a patient, it is preferably administered usually 1 to 1000 mg or, preferably, 5 to 200 mg per day for an adult by dividing into one to three times.

The ACAT inhibitory action, etc. of the compound of the present invention has been tested by a method mentioned in the following examples.

## Examples

As hereunder, the present invention will be more specifically illustrated by the following Examples although the present invention is not limited by those Examples at all.

Example 1

Method for ACAT inhibitory activity test in J774 cells and HepG2 cells (Anti-foaming action)

J774 cells or HepG2 cells were sown on a 24-well plate and incubation was carried out at 37°C for 24 hours in a 5% $CO_2$ incubator using a Dulbecco's modified Eagle's medium for J774 cells and an Eagle's minimum essential medium for HepG2 cells (where both media contained 10% of fetal bovine serum). After exchanging to 0.5 mL each of a culture solution containing 10 μg/mL of 25-hydroxycholesterol and a sample to be tested, incubation was carried out for 18 hours more. The medium was taken out, washed with PBS twice, extracted with 1.5 mL of hexane-isopropanol (3:2) and concentrated to dryness. The extract was dissolved in isopropanol containing 0.2 mL of 10% Triton X-100 and then total cholesterol (TC) and free cholesterol (FC) were determined by a Cholesterol E Test Wako (Wako Pure Chemical) and a Free Cholesterol E Test Wako (Wako Pure Chemical), respectively. The residue of the cell after extraction was solubilized with 0.25 mL of 2N NaOH at 37°C for 30 minutes and protein was determined by a BCA Protein Assay Reagent (Pierce). From the differencebetween TC and FC, amount of cholesterolester per protein was calculated and, from the comparative calculation with the control, $IC_{50}$ value was determined.

Example 2

Test method for oral absorption in rats

A solution of the compound of the present invention (dissolved in a mixture of water : PEG 400 = 1:4) or a suspension in 0.5% methyl cellulose (MC) was compulsorily administered *per os* at. the dose of 30 mg/kg to three rats under a fed condition. Blood after the administration was periodically collected and its concentration in plasma was measured. At the same time, a solution of the compound of Example 85 of the Japanese Patent Application No. 11/500,471 (dissolved in PEG 400) was compulsorily administered *per os* at the dose of 30 mg/kg and its concentration in plasma was measured.

Example 3

Measuring method for the concentration of the compound of the present invention in plasma

To 150 μL of plasma were added a 1 mol/L glycine buffer (pH 8) and 0.2 mL of an analogous compound (500 ng/mL of the compound of Example 98 of the Japanese Patent Application No. 11/500,471, i.e. 2-[4-[2-(4,5,6-trimethoxybenzothiazol-2-ylthio)ethyl]piperazin-1-yl]-N-(2,6-diisopropylphenyl)-acetamide), as an internal standard, stirred and extracted with 6 mL of an isopropanol/n-hexane (5:95) solution. The organic layer (upper layer) was collected, the solvent was evaporated in a nitrogen stream, the residue was dissolved in 0.2 mL of a mobile phase (refer to the following) and 80 μL thereof were measured under the following analytical conditions.

Condition for detection:

(Detector: Single-stage quadrupole mass spectrometer
Ionization method: Atmospheric pressure chemical ionization method
Temperature of nebulizer: 475°C
Corona voltage: 3 kV
Monitoring ion: m/z 565 (hydrogen addition ion of the compound of the present invention) , 579 (hydrogen addition ion of the compound of Example 98 of the Japanese Patent Application No. 11/500,471))

Condition for separation:

(Column: Inertsil ODS 3V manufactured by GL Science (3.0 mmϕ × 150 mm)
Temperature of column: 45°C
Mobile phase: a solution where 0.1% ammonium formate (pH 4)/methanol/acetonitrile are mixed in 7:3:10
Flow rate: 1.0 mL/min)

Example 4

Measuring method for the compound of Example 78 of the Japanese Patent Application No. 11/500,471 in plasma

To 150 μL of plasma were added a 0.1 mol/L aqueous solution of sodium hydroxide and 0.2 mL of an analogous

compound (500 ng/mL) Example 85 of the Japanese Patent Application No. 11/500,471, i.e. 2-[4-[2-(7-trifluoromethylbenzoxazol-2-ylthio)ethyl]piperazin-1-yl]-N-(2,6-diisopropylphenyl)-acetamide as an internal standard, stirred and extracted with 6 mL of an isopropanol/hexane (5:95) solution. The organic layer was collected, the solvent was evaporated in a nitrogen stream, the residue was dissolved in 0.2 mL of a mobile phase (refer to the following) and 80 μL thereof were measured under the following condition for high-performance liquid chromatography.

Condition for detection:

(Detector: Single-stage quadrupole mass spectrometer
Ionization method: Atmospheric pressure chemical ionization method
Temperature of nebulizer: 475°C
Corona voltage: 3 kV
Monitoring ion: m/z 569 (hydrogen addition ion of the compound of Example 78), 549 (hydrogen addition ion of the compound of Example 85))

Condition for separation:

(Column: Symmetry RP8 manufactured by Waters (3.0 mmϕ × 150 mm)
Temperature of column: 45°C
Mobile phase: a solution where 0.1% ammonium formate (pH 4)/methanol/acetonitrile are mixed in 7:.3:10
Flow rate: 0.8 mL/min)

Example 5

Measuring method for the compound of Example 85 of the Japanese Patent Application No. 11/500,471 in plasma

To 150 μL of plasma were added a 0.1 mol/L aqueous solution of sodium hydroxide and 0.2 mL of an analogous compound (500 ng/mL of the compound of Example 28 of the Japanese Patent Application No. 8/158,743, i.e. 8-(benzimidazol-2-ylthio)-N-(2,6-diisopropylphenyl)octanamide as an internal standard, stirred and extracted with 6 mL of an isopropanol/hexane (5: 95) solution. The organic layer was collected, the solvent was evaporated in a nitrogen stream, the residue was dissolved in 0.2 mL of a mobile phase (refer to the following) and 80 μL thereof were measured under the following condition for high-performance liquid chromatography.

Condition for detection:

(Detector: Single-stage quadrupole mass spectrometer
Ionization method: Atmospheric pressure chemical ionization method
Temperature of nebulizer: 475°C
Corona voltage: 3 kV
Monitoring ion: m/z 549 (hydrogen addition ion of the compound of Example 85), 452 (hydrogen addition ion of the compound of Example 28))

Condition for separation:

(Column: Symmetry RP8 manufactured by Waters (3.0 mmϕ × 150 mm)
Temperature of column: 45°C
Mobile phase: a solution where 0.1% ammonium formate (pH 4)/methanol/acetonitrile are mixed in 7:3:10
Flow rate: 0.8 mL/min)

Example 6

Test method for arteriosclerotic model animals

In a test using arteriosclerotic model animal which was a lipid-loaded model of $F_1B$ hamster according to a method by Robert, et al., the area where lipid was accumulated in aortic arch was measured and the suppressing effect of the compound of thepresent invention for lipid accumulation was investigated [Refer to Robert, J. N., et al., *Atherosclerosis*, 137, 77-85 (1988)]. As control drugs, the compound of Example 24 showing the strongest ACAT inhibitory activity in the Japanese Patent Application No. 11/500,471 and the already-reported CI-1011 (2,6-diisopropylphenyl N-[2-(2,4,6-triisopropylphenyl)acetyl]sulfamate (Example 5 of the JP-T-8-510,256 and WO 94/26702) were used.
Male hamsters of a Bio $F_1B$ strain purchased from Nippon Charles River were randomly grouped so that each

group comprised six. As from eight weeks age, they were loaded with a high-fat feed (0.3% of cholesterol and 10% of coconut oil) for ten weeks while the drug was administered by mixing with the feed. After ten weeks, blood was collected and cholesterol in plasma was measured. Further, arterywas excised after fixingbyperfusion with a 10% formalin solution. This was incised, lipid which was accumulated in the artery was stained with Oil Red O and the stained area was measured using an image analyzer.

Methods for preparation and administration of the drug

A high-fat feed (0.3% of cholesterol and 10% of coconut oil) was prepared by way of compounding ratio of cholesterol : coconut oil : feed (CE-2) = 0.03:1:9. Incidentally, a high-fat feed was given to the control group and the administered period was 10 weeks. Setting of the dose was that, as a result of the preliminary investigation, administration of 100 mg/kg was the upper limit and, as a common ratio of 3 from the dose where decrease of total cholesterol in plasma was not noted, there were set four doses.

Constitutions of the groups

Control group (high-fat feed) : (n = 6)
Groups fed with 3, 10, 30 and 100 mg/kg of the compound of the present invention: (n = 6 in each group)
Groups fed with 1, 3, 10 and 30 mg/kg of the compound of Example 24: (n = 6 in each group)
Groups fed with 0.3, 1, 3 and 10 mg/kg of CI-1101: (n = 6 in each group)

Methods for observation and test

Concentrations of lipid in blood and in tissues (liver, small intestine and adrenal body)

Hamster under a non-fasted state was anesthetized, subj ected to an abdominal section to expose the abdominal artery and about 2.5 mL of blood were collected. The collected blood was centrifuged at 3, 000 rpm for 15 minutes to collect the plasma and then total cholesterol (TC) and free cholesterol (FC) in plasma were determined by a Cholesterol E-Test Wako (a cholesterol oxidase DAOS method) and a Free Cholesterol E-Test Wako (a cholesterol oxidase DAOS method), respectively.
About 500 mg of liver, small intestine and adrenal body were excised and washed with a physiological saline solution and lipid concentration in the tissues was measured. In the measurement, 10 mL of chloroform : methanol (2: 1) as an extracting solution for lipid in the tissue were placed in the liver and the small intestine, homogenized at 3,000 rpm using a Polytron Homogenizer PT 3100 and allowed to stand at 4°C for one night. The extract was centrifuged at 1,500 rpm for 15 minutes, 0.2 mL of the supernatant liquid was evaporated to dryness using a vacuum pump, the residue was dissolved in 50 μL of isopropanol and each lipid was subjected to the same kits whereupon TC and FC were quantified. With regard to the adrenal body, it was finely cut using scissors and 0.5 mL of chloroform : methanol (2:1) was added thereto followed by being allowed to stand at 4°C for one night. That was centrifuged at 1,500 rpm for 15 minutes, 0.2 mL of the supernatant liquid was evaporated to dryness using a vacuum pump, the residue was dissolved in 50 μL of isopropanol and each lipid was subjected to the same kits whereupon TC and FC were quantified.
With regard to the total cholesterol (TC) in plasma in the group to which the drug was administered, the relative ratio to TC in the control group was used as an indicator while, with regard to the tissue, difference between TC and FC was defined as CE (cholesterol ester) and the lipid-constituting ratio, CE/FC, in each organ was determined whereupon their relative ratio to each control was calculated and used as an indicator. Thus, in those indicators, the control group was defined as 100% and the calculation using the following formula was carried out so as to use a plasma TC lowering action by ACAT inhibitory action to liver and small intestine of the group to which the drug was administered as an indicator and also to use the variation of CE/FC ratio by an ACAT inhibitory action to each organ as an indicator.

Indicator (%) for total cholesterol in plasma = [(TC of

drug-administered group)/(TC of the control group)] × 100

Indicator (%) for lipid constituting ratio in each organ

= [(CE/FC of drug-administered group)/(CE/FC of the control

group)] × 100

Lipid Accumulation Area in Artery

After collection of the blood, a 18-G injection needle was inserted into apex and perfused with a physiological saline solution (120 mmHg) for about 5minutes. It was further perfused with a 10% neutral formalin buffer (120 mmHg) for about 5 minutes. Bulbar and thoracic artery was excised and fixed in a 10% formalin buffer. After being fixed, lesser curvature of artery and a part of greater curvature were opened and stained with Oil Red O. That was opened and adhered on a rubber plate and area of the part stained with Oil Red O and area of the surface of lumen were measured by an Olympus Image Analyzer. In each example of each group, the ratio of area stained with Oil Red O to area of lumen surface was determined and defined as artery lipid accumulation rate. With regard to artery lipid accumulation suppressing rate, the ratio of artery lipid accumulation rate of the drug-administered group to artery lipid accumulation rate of the control group was determined and that was used as an indicator for artery accumulation suppressing rate by an ACAT suppressing action.

Indicators were calculated by the following formulae.

Artery lipid accumulation rate (%) = [(Area of the part stained with Oil Red O)/(area of lumen surface)] × 100

Artery lipid accumulation suppressing rate (%) = [(Artery lipid accumulation rate in each group)/(Artery lipid accumulation rate in control group)] × 100

Example 7

Example of production

Production of tert-butyl 4-[2-(7-trifluoromethylbenzoxazol-2-ylthio)ethyl]-1-piperazinecarboxylate

Triethylamine (17.14 g, 0.169 mol) and 4-dimethylaminopyridine (1.59 g, 13.0 mmol) were added to a solution of tert-butyl 4-(2-hydroxyethyl)-1-piperazinecarboxylate (30.00 g, 0.130 mol) in THF (200 mL) and a solution of methanesulfonyl chloride (17.91 g, 0.156 mol) in THF (100 mL) was dropped thereinto with ice-cooling followed by stirring for 30 minutes. The reaction solution was filtered and the filtrate was concentrated *in vacuo*. The resulting residue was dissolved in DMF (300 mL), dropped into a solution of 2-mercapto-7-trifluoromethylbenzoxazole (28.55 g, 0.130 mol) prepared by a method mentioned in Example 85 of the Japanese Patent Application No. 11/500,471, 18-crown-6(1.72 g, 6.51 mmol) and potassium carbonate (21.60 g, 0.156 mol) in DMF (500 mL) at 80°C followed by stirring at the same temperature for 30 minutes. The reaction solution was concentrated *in vacuo* and the resulting residue was extracted with water and ethyl acetate. The organic layer was washed with water and a saturated saline solution, dried over anhydrous sodium sulfate and concentrated *in vacuo*. The resulting residue was purified by a silica gel column chromato graphy (silica gel: 600g; developing solvent: hexane/acetone = 5/1) to give 39.82 g (yield: 71%) of an aimed compound as a pale brown oily product.

IR (neat) $cm^{-1}$: 2977, 1699, 1506, 1493, 1432

$^1$H-NMR ($CDCl_3$) δ: 1.46 (9H, s), 2.49 (4H, t, J = 4.9 Hz), 2.83 (2H, t, J = 6.8 Hz), 3.43 (4H, t, J = 4.9 Hz), 3.49 (2H, t, J = 6.8 Hz), 7.37 (1H, t, J = 8.1 Hz), 7.47 (1H, d, J = 8.1 Hz), 7.75 (1H, d, J = 8.1 Hz)

| Elementary analysis as $C_{19}H_{24}F_3N_3O_3S$: | | | | |
|---|---|---|---|---|
| Calculated | C, 52.89; | H, 5.61; | N, 9.74; | F, 13.21 |
| Found | C, 53.07; | H, 5.69; | N, 9.76; | F, 13.11 |

Example 8

Production of 1-[2-(7-trifluoromethylbenzoxazol-2-yl-thio)ethyl]piperazine ditrifluoroacetate

tert-Butyl 4-[2-(7-trifluoromethylbenzoxazol-2-yl-thio)ethyl]-1-piperazinecarboxylate (37.92 g, 87.9 mmol) was dissolved in trifluoroacetic acid (200 mL) under ice-cooling and stirred at the same temperature for 15 minutes. Ether was added to the reaction solution under ice-cooling and the separated crystals were filtered, washed with ether and dried *in vacuo* to give 47.46 g (yield: 97%) of the aimed compound as light yellow powdery crystals.

Melting point: 155-156°C

IR (KBr) $cm^{-1}$: 3026, 2421, 1683, 1511, 1596

$^1$H-NMR ($d_6$-DMSO) δ: 2.75-2.90 (4H, m) , 2.91-3.04 (2H, m), 3.05-3.22 (4H, m), 3.56 (2H, t, J = 6.8 Hz), 7.54 (1H, t, J = 8.0 Hz), 7.67 (1H, d, J = 8.0 Hz), 7.96 (1H, d, J = 8.0 Hz), 8.70 (1H, br s).

| Elementary analysis as $C_{18}H_{18}F_9N_3O_5S$: | | | |
|---|---|---|---|
| Calculated | C, 38.65; | H, 3.24; | N, 7.51 |
| Found | C, 38.60; | H, 3.25; | N, 7.51. |

Example 9

Production of 2-bromo-N-(2,6-diisopropyl-4-hydroxyphenyl)acetamide

A solution of N,N-dimethylaniline (6.60 g, 54.5 mmol) in methylene chloride (100 mL) was dropped into a solution of 4-amino-3,5-diisopropylphenol (synthesized by a method mentioned in Example 72 of the Japanese Patent Application No. 11/500,471) (8.77 g, 45.4 mmol) in methylene chloride (100 mL) with ice-cooling and stirring. After that, a solution of bromoacetyl bromide (10.1 g, 49.9 mmol) in methylene chloride (75 mL) was gradually dropped thereinto followed by stirring at room temperature for 30 minutes. The solvent was evaporated until the reaction solution became about 100 mL, water (100 mL) and a saturated aqueous solution of sodiumhydrogencarbonate (100 mL) were added thereto and the mixture was cooled with ice for about 2 hours. The separated crystals were filtered and washed with 1N hydrochloric acid. The resulting crystals were dissolved in chloroform (100 mL) and methanol (25 mL) and washed with 1N hydrochloric acid for three times. The organic layer was further washed with water and a saturated aqueous saline solution, dried over anhydrous sodium sulfate and concentrated *in vacuo* to give 12.28 g (yield: 86%) of the aimed compound as brown needles.

Melting point: 196-197°C

IR (KBr) $cm^{-1}$: 3288, 2966, 1665, 1593, 1534.

$^1$H-NMR ($CDCl_3$) δ: 1.18 (12H, d, J = 6.8 Hz), 2.98 (2H, sept, J = 6.8 Hz), 4.08 (2H, s), 6.64 (2H, s), 7.57 (1H, br s).

EIMS m/z (relative intensity): 315 ($M^+$ + 1), 313 ($M^+$-1), 220 (100).

| Elementary analysis as $C_{14}H_{20}BrNO_2$: | | | |
|---|---|---|---|
| Calculated | C, 53.51; | H, 6.42; | N, 4.46 |
| Found | C, 53.50; | H, 6.33; | N, 4.28 |

Example 10

Production of 2-[4-[2-(7-trifluoromethylbenzoxazol-2-ylthio)ethyl]piperazin-1-yl]-N-(2,6-diisopropyl-4-hydroxyphenyl) acetamide hydrochloride (compound of the present invention)

Sodium hydrogencarbonate (24.03 g, 0.286 mol) was added to a suspension of 1-[2-(7-trifluoromethylbenzoxazol-2-yl-thio) ethyl]piperazine ditrifluoroacetate (40.00 g, 71.5 mmol) in acetonitrile (1 L) at room temperature and stirred at the same temperature for 1 hour. 2-Bromo-N-(2,6-diisopropyl-4-hydroxyphenyl)acetamide (22.47 g, 71.5 mmol) and potassium carbonate (14.82 g, 0.107 mol) were successively added to the reaction solution under ice-cooling and stirred at room temperature for 20 hours. The reaction solution was concentrated in vacuo and the resulting residue was extracted with water and chloroform. The organic layer was washed with a saturated aqueous saline solution, dried over anhydrous sodium sulfate and dried *in vacuo.* The resulting residue was purified by a silica gel column chromatography (silica gel: 400 g; developing solvent: hexane/acetone = 2/1 → 1/1) and recrystallized from acetone-hexane to give 39.37 g (yield: 98%) of 2-[4-[2-(7-trifluoromethylbenzoxazol-2-ylthio)ethyl]-piperazin-1-yl] -N-(2,6-diisopropyl-4-hydroxyphenyl)acetamide in a free form as light yellow powdery crystals.

A solution of 4N hydrogen chloride in ethyl acetate (16.9 mL, 67.6 mmol) was dropped into a methanolic solution (150 mL) of the free amide (34.71 g, 61.5mmol) under ice-cooling followed by stirring at the same temperature for 10 minutes. Ether was added to the reaction solution at the same temperature and the separated crystals were filtered, washed with ether and dried *in vacuo* to give 34.35 g (yield: 93%) of the aimed compound as colorless powdery crystals.

Melting point: 258-259°C

IR (KBr) $cm^{-1}$: 3440, 2967, 1661, 1609, 1594

$^1$H-NMR ($d_6$-DMSO, 120°C) δ: 1.12 (12H, d, J = 6.8 Hz), 2.97 (2H, sept, J = 6.8 Hz), 3.05-3.24 (10H, m), 3.66 (2H, t, J = 6.8 Hz), 3.78 (2H, s), 6.57 (2H, s), 7.54 (1H, t, J = 7.8 Hz), 7.63 (1H, d, J = 7.8 Hz), 7.92 (1H, d, J = 7.8 Hz), 9.10 (1H, br s)

| Elementary analysis as $C_{28}H_{36}ClF_3N_4O_3S$: | | | | | |
|---|---|---|---|---|---|
| Calculated | C, 55.95; | H, 6.04; | N, 9.32; | Cl, 5.90; | F, 9.48 |
| Found | C, 55.80, | H, 6.01; | N, 9.23; | Cl, 5.92; | F, 9.31 |

Example 11

Production of 2-[4-[2-(7-trifluoromethylbenzoxazol-2-ylthio)ethyl]piperazin-1-yl]-N-(2,6-diisopropyl-3-hydroxyphenyl) acetamide (Comparative compound)

2-[4-[2-(7-trifluoromethylbenzoxazol-2-ylthio)ethyl] piperazin-1-yl]-N-(2,6-diisopropyl-3-nitrophenyl)acetamide prepared in Example 87 of the Japanese Patent Application No. 11/500, 471 was reduced according to amethodmentioned in Example 59 of the same patent specification and the resulting 2-[4-[2-(7-trifluoromethylbenzoxazol-2-ylthio)ethyl]-piperazin-1-yl]-N-(3-amino-2,6-diisopropylphenyl)acetamide was made to react by the same method mentioned in Example 61 of the same patent specification to give an aimed compound as colorless crystals.

Melting point: 82-84°C

IR (KBr) $cm^{-1}$: 3314, 1667, 1595, 1505, 1330

$^1$H-NMR ($CDCl_3$) δ: 1.16 (6H, d, J = 6.8 Hz), 1.34 (6H, d, J = 6.8 Hz), 2.60-2.77 (8H, m), 2.85 (2H, t, J= 6.8 Hz), 2.89 (1H, sept, J = 6.8 Hz), 3.14 (1H, sept, J= 6.8 Hz), 3.20 (2H, s), 3.50 (2H, t, J = 6.8 Hz), 5.82 (1H, br s), 6.65 (1H, d, J = 8.5 Hz), 6.99 (1H, d, J = 8.5 Hz), 7.34 (1H, t, J = 7.8 Hz), 7.47 (1H, d, J = 7.8 Hz), 7.75 (1H, d, J = 7.8 Hz), 8.58 (1H, br s).

EIMS m/z (relative intensity): 564 ($M^+$), 346 (100).

Industrial Applicability

The present invention provides a novel compound which retains anACAT inhibitoryactivityandamacrophage selectivity to some extent as an actual drug and has excellent oral absorption property, a process for producing the same, a pharmaceutical composition using the same and an intermediate therefor.

The compound of the present invention has an excellent oral absorption, is able to retain a high concentration in blood and has a very good advantageous effects as an ACAT inhibitory agent in view of vascular selectivity and it is a substance having a very good selectivity as an effective ingredient of drugs.

## Claims

**1.** 2-[4-[2-(7-trifuoromethylbenzoxazol-2-ylthio)-ethyl]piperazin-1-yl]-N-(2,6-diisopropyl-4-hydroxyphenyl)-acetamide represented by the following formula, a salt thereof or a solvate thereof.

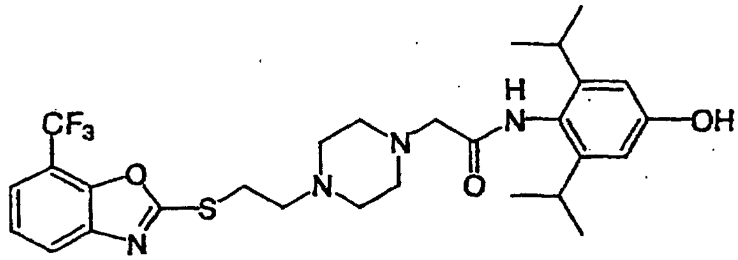

2. Apharmaceutical composition having an ACAT inhibitory action which comprises the compound represented by the following formula, a salt thereof or a solvate thereof and a pharmaceutically acceptable carrier.

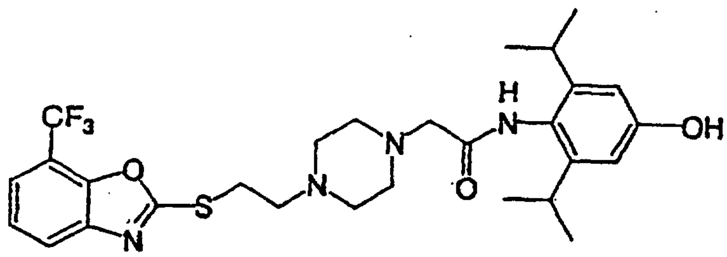

3. A compound represented by the following formula or a salt thereof.

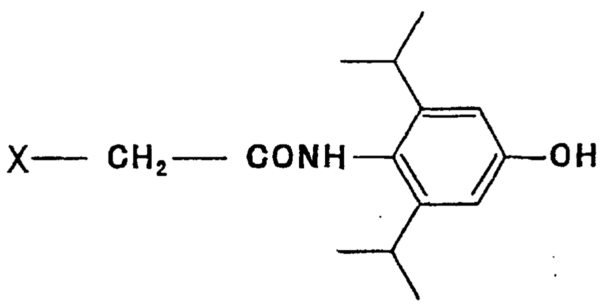

(in the formula, X is a halogen atom).

Figure 1

Compound of the present invention

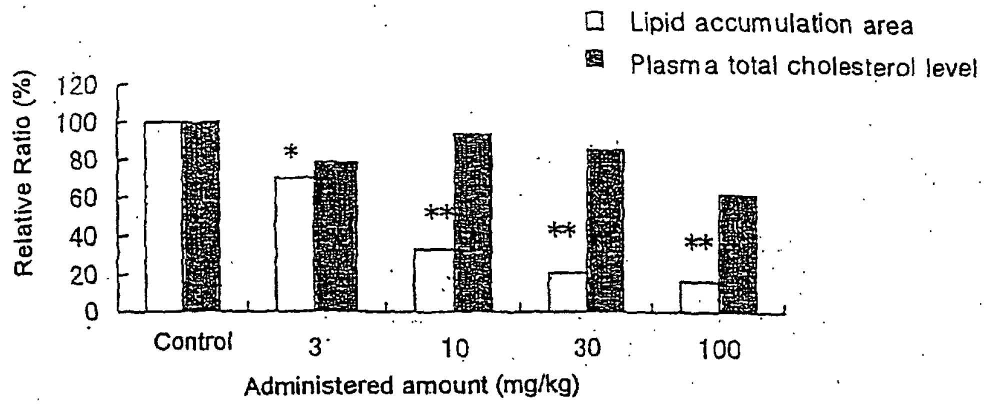


Figure 2

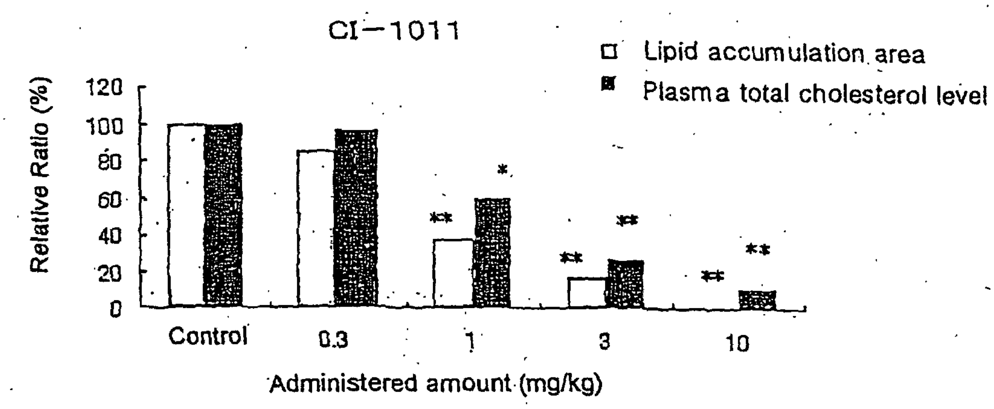


Figure 3

Compound of Example 24

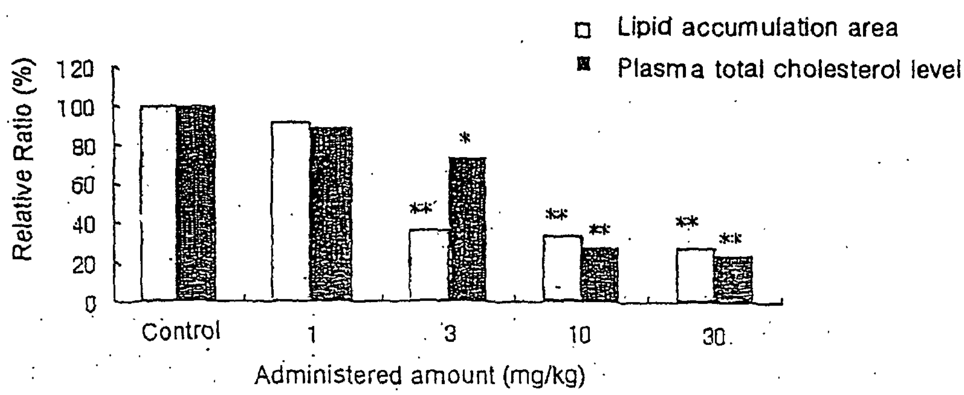

Figure 4

Compound of the present invention

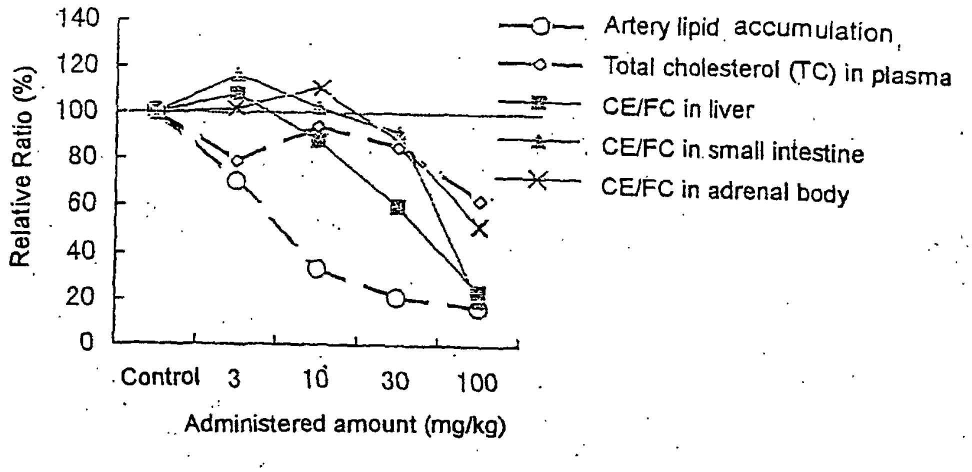


Figure 5

CI−1011

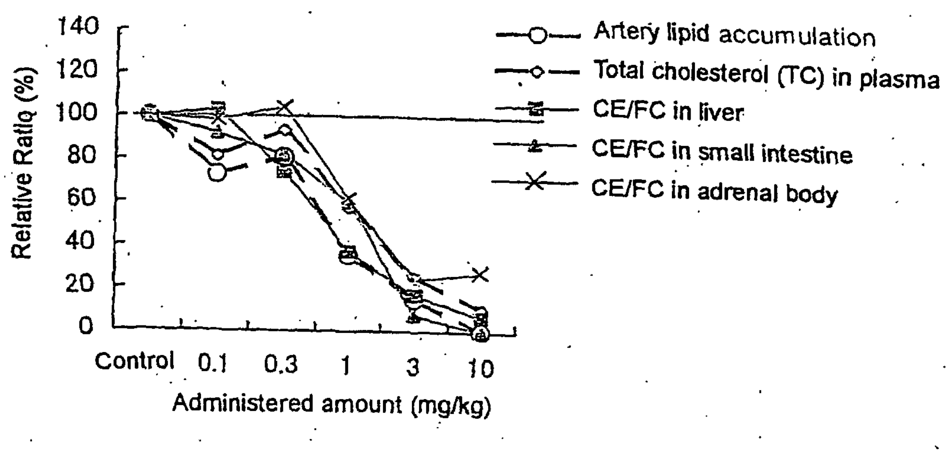


Figure 6

Compound of Example 24

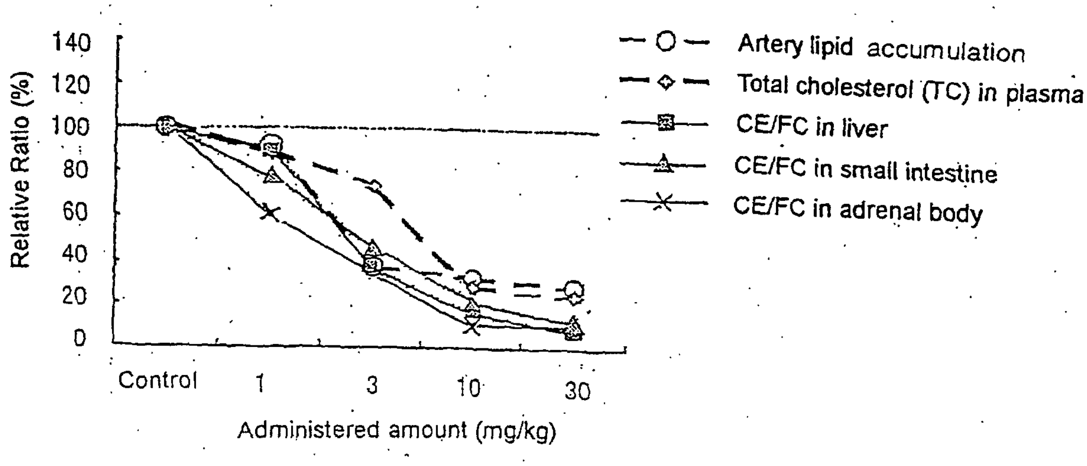

## INTERNATIONAL SEARCH REPORT

International application No.
PCT/JP02/08343

A. CLASSIFICATION OF SUBJECT MATTER
Int.Cl[7] C07D263/58, A61K31/496, A61P3/06, 9/10, 43/00, C07C233/25

According to International Patent Classification (IPC) or to both national classification and IPC

B. FIELDS SEARCHED

Minimum documentation searched (classification system followed by classification symbols)
Int.Cl[7] C07D263/58, A61K31/496, A61P3/06, 9/10, 43/00, C07C233/25

Documentation searched other than minimum documentation to the extent that such documents are included in the fields searched

| | | | |
|---|---|---|---|
| Jitsuyo Shinan Koho | 1992-1996 | Jitsuyo Shinan Toroku Koho | 1996-2002 |
| Kokai Jitsuyo Shinan Koho | 1971-2002 | Toroku Jitsuyo Shinan Koho | 1994-2002 |

Electronic data base consulted during the international search (name of data base and, where practicable, search terms used)
REGISTRY(STN), CAPLUS(STN)

C. DOCUMENTS CONSIDERED TO BE RELEVANT

| Category* | Citation of document, with indication, where appropriate, of the relevant passages | Relevant to claim No. |
|---|---|---|
| X | EP 987254 A1 (Kowa Co., Ltd.), 22 March, 2000 (22.03.00), Claims; examples 48, 69, 72, 85 & WO 98/54153 A1 | 1-3 |
| X | EP 807627 A2 (Kowa Co., Ltd.), 19 November, 1997 (19.11.97), Full text JP 10-29986 A & US 6204278 B1 | 1-3 |

☐ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 09 October, 2002 (09.10.02) | 29 October, 2002 (29.10.02) |
| Name and mailing address of the ISA/ Japanese Patent Office | Authorized officer |
| Facsimile No. | Telephone No. |

Form PCT/ISA/210 (second sheet) (July 1998)